(19) Europäisches Patentamt
European Patent Office
Office européen des brevets

(11) Veröffentlichungsnummer: **0 001 064**
**B1**

(12) **EUROPÄISCHE PATENTSCHRIFT**

(45) Veröffentlichungstag der Patentschrift: **07.07.82**

(21) Anmeldenummer: **78100708.3**

(22) Anmeldetag: **21.08.78**

(51) Int. Cl.³: **C 07 C 69/743**, C 07 C 69/612, C 07 C 121/75, C 07 C 43/257, C 07 D 317/60, A 01 N 31/14, A 01 N 37/10, A 01 N 53/00

(54) **Fluorsubstituierte Phenoxybenzyl-oxycarbonylderivate,-alkohole und -halogenide, Verfahren zu ihrer Herstellung und ihre Verwendung.**

(30) Priorität: **03.09.77 DE 2739854**

(43) Veröffentlichungstag der Anmeldung: **21.03.79 Patentblatt 79/6**

(45) Bekanntmachung des Hinweises auf die Patenterteilung: **07.07.82 Patentblatt 82/27**

(84) Benannte Vertragsstaaten: **BE CH DE FR GB NL SE**

(56) Entgegenhaltungen:
**FR-A-2 289 484**
**FR-A-2 360 567**
**US-A-3 666 789**

**AGRICULTURE AND BIOLOGICAL CHEMISTRY, vol. 40, Nr. 1, 1976, TAKASHI MATSUO et al. «3-Phenoxy-α-cyano-benzyl Esters, the most potent synthetic pyrethroids», Seiten 247–249**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Hammann, Ingeborg, Dr., Belfortstrasse 9, D-5000 Köln 1 (DE)**
Erfinder: **Behrenz, Wolfgang, Dr., Untergruendemich 14, D-5063 Overath (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 001 064 B1

ACTORUM AG

# Fluorsubstiuierte Phenoxybenzyl-oxycarbonylderivate, -alkohole und -halogenide, Verfahren zu ihrer Herstellung und ihre Verwendung

Die vorliegende Erfindung betrifft neue fluorsubstituierte Phenoxybenzyl-oxycarbonylderivate, -alkohole und -halogenide, Verfahren zu ihrer Herstelung und ihre Verwendung.

Es ist bereits bekannt, dass Phenoxybenzyl-acetate oder -carboxylate, wie z.B. 3'Phenoxybenzyl-α-isopropyl-(3,4-dimethoxyphenyl)-acetat und 3'-[2-Fluor- bzw. 4-Fluorphenoxy]-α-cyanobenzyl-[2,2-dimethyl-3-(2,2-dichlorvinyl)-cyclopropan]-carboxylat, insektizide und akarizide Eigenschaften besitzen (vgl. Deutsche Offenlegungsschriften 2 335 347 und 2 547 534 und Belgische Patentschrift 801 946).

Es wurden nun die neuen fluorsubstituierten Phenoxybenzyl-oxycarbonylderivate, -alkohole und -halogenide der Formel (I)

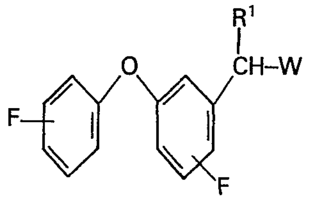

(I)

gefunden, in welcher

W für OH, Halogen oder den Rest $-OCO-R^2$ steht und

$R^1$ für Wasserstoff, Cyan oder Äthinyl steht und

$R^2$ für den Rest

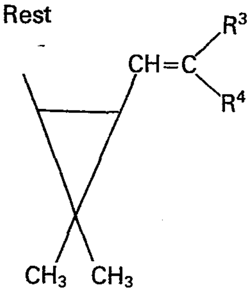

steht, wobei

$R^3$ und $R^4$ gleich sind und für Chlor, Brom oder Methyl stehen und ferner

$R^2$ für den Rest

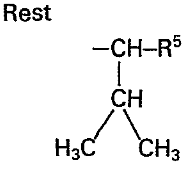

steht, wobei

$R^5$ für einen gegebenenfalls durch Halogen, Alkyl, Alkylthio bzw. Alkoxy mit jeweils 1–4 Kohlenstoffatomen, Nitro oder Methylendioxy substituierten Phenylring steht.

Es wurde ferner ein Verfahren zur Herstellung der fluorsubstituierten Phenoxybenzyl-oxycarbonylderivate, -alkohole und -halogenide der Formel (I) gefunden, das dadurch gekennzeichnet ist, dass man für den Fall, dass W für den Rest $-O-CO-R^2$ steht,

a) Carbonylhalogenide der Formel (II)

$$Hal-CO-R^2 \qquad (II)$$

in welcher

$R^2$ die angegebene Bedeutung hat und

Hal für Halogen, vorzugsweise Chlor, steht,

mit fluorsubstituierten Phenoxybenzylalkoholen der Formel (III)

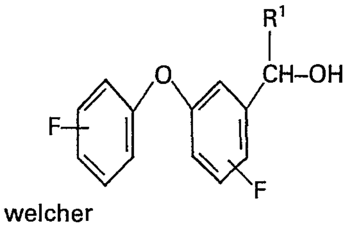

(III)

in welcher

$R^1$ die angegebene Bedeutung hat,

gegebenenfalls in Gegenwart eines Säureakzeptors und gegebenenfalls in Gegenwart eines Lösungsmittels, oder

b) Carboxylderivate der Formel (IV)

$$HO-CO-R^2 \qquad (IV)$$

in welcher

$R^2$ die angegebene Bedeutung hat,

gegebenenfalls in Form der Alkali–, Erdalkali– oder Ammoniumsalze oder gegebenenfalls in Gegenwart eines Säureakzeptors mit fluorsubstituierten Phenoxybenzylhalogeniden der Formel (V)

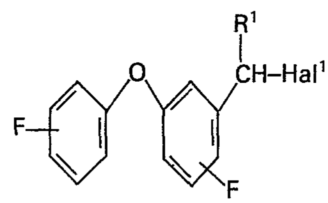

(V)

in welcher

$R^1$ die angegebene Bedeutung hat, und

$Hal^1$ für Halogen, vorzugsweise Brom, steht,

gegebenenfalls in Gegenwart eines Lösungsmittels umsetzt, und ferner für den Fall, dass W für OH oder Halogen steht, in an sich bekannter Weise die entsprechenden Phenoxytoluole mit Halogenierungsmitteln in die entsprechenden Phenoxybenzylhalogenide überführt, diese mit Hexamethylentetramin zu den entsprechenden Phenoxybenzyldehyden umsetzt und diese

a[1]) für den Fall, dass $R^1$ für Wasserstoff steht, mit einem komplexen Metallhydrid in einem inerten Lösungsmittel reduziert,

b[1]) für den Fall, dass $R^1$ für Cyan steht, mit einem Alkalimetallcyanid in Gegenwart einer Säure gegebenenfalls unter Zusatz eines Lösungsmittels umsetzt, oder

c[1]) für den Fall, dass $R^1$ für Äthinyl steht, mit einer Äthinylverbindung der Formel

$$H-C-C{\equiv}Mg-Hal$$

in welcher

Hal für Halogen steht, in einem geeigneten Lösungsmittel umsetzt.

Flurosubstituierte Phenoxybenzyloxycarbonylderivate der Formel

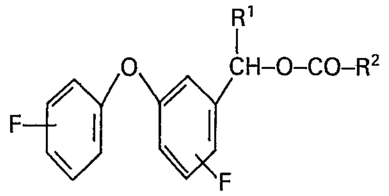

in welcher

$R^1$ für Wasserstoff, Cyan oder Äthinyl steht und

$R^2$ für den Rest

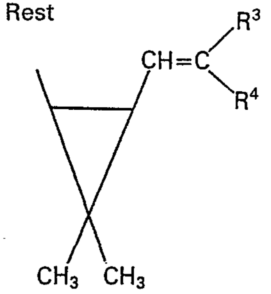

wobei

$R^3$ und $R^4$ gleich sind und für Chlor, Brom oder Methyl stehen oder für den Rest

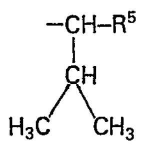

steht, wobei

$R^5$ für einen gegebenenfalls durch Halogen, Alkyl, Alkylthio bzw. Alkoxy mit jeweils 1–4 Kohlenstoffatomen, Nitro oder Methylendioxy substituierten Phenylring steht,

zeichnen sich durch eine starke insektizide und akarizide Wirksamkeit aus.

Die allgemeine Formel schliesst dabei die verschiedenen möglichen Stereoisomeren, die optischen Isomeren und Mischungen dieser Komponenten ein.

Überraschenderweise zeigen diese erfindungsgemässen fluorsubstituierten Phenoxybenzyloxycarbonylderivate eine bessere insektizide und akarizide Wirkung als die entsprechenden vorbekannten Produkte analoger Konstitution und gleicher Wirkungsrichtung. Die Produkte gemäss vorliegender Erfindung stellen somit eine echte Bereicherung der Technik dar.

Verwendet man beispielsweise 2-Fluor-5-(3-fluorphenoxy)-α-Cyan-benzylalkohol bzw. -bromid und α-Isopropyl-4-äthylphenylessigsäurechlorid oder -essigsäurenatriumsalz als Ausgangsmaterialien, so kann der Reaktionsverlauf durch die folgenden Formelschemata wiedergegeben werden:

a) 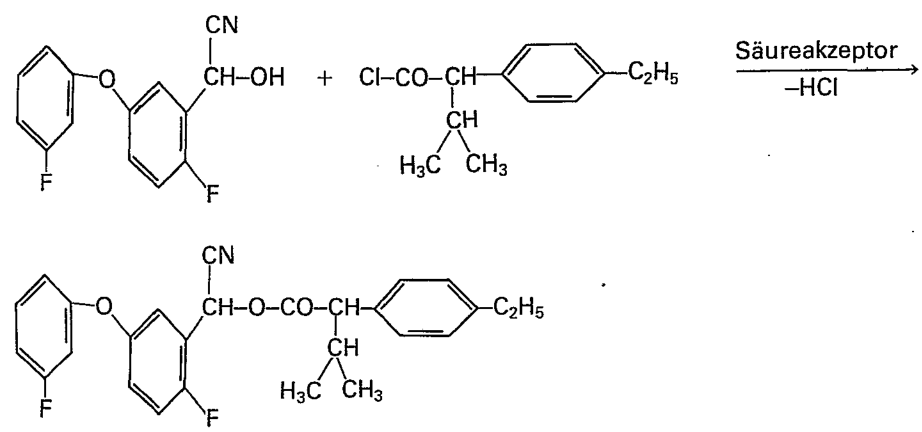


b) 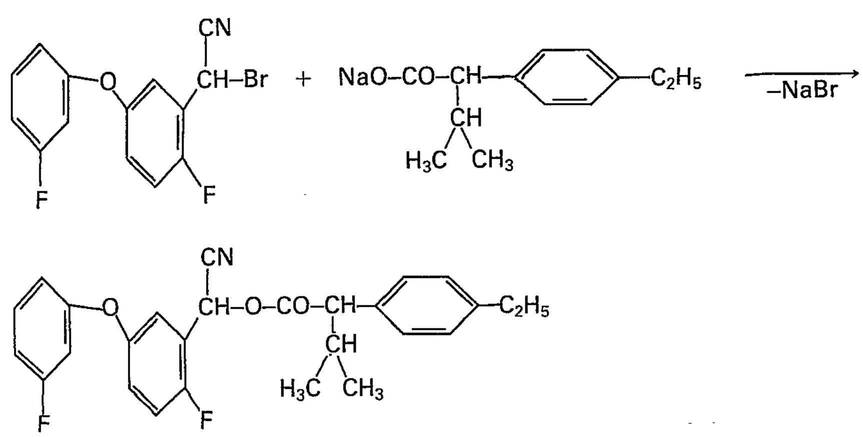


Die zu verwendenden Ausgangsstoffe sind durch die Formeln (II) bis (V) eindeutig allgemein definiert. Vorzugsweise stehen darin jedoch

$R^1$ für Wasserstoff oder Cyan und

$R^2$ für den 2,2-Dimethyl-3-(2,2-dichlor- bzw. 2,2-dibrom- und 2,2-dimethyl-vinyl)-cyclopropanrest,

für den α-Isopropylbenzylrest, der gegebenenfalls im Ring ein- oder mehrfach, gleich oder verschieden durch Fluor, Chlor, Brom und/oder geradkettiges oder verzweigtes Alkyl mit 1 bis 3 Kohlenstoffatomen substituiert sein kann.

Die als Ausgangsprodukte zu verwendenden Carbonylhalogenide (II) bzw. Carboxylderivate (IV) sind teilweise bekannt oder können nach allgemein üblichen, in der Literatur beschriebenen Verfahren hergestellt werden (vergleiche z.B. Deutsche Offenlegungsschrift 2 365 555; 1 926 433 und 2 231 312).

Als Beispiele hierfür seien im einzelnen genannt:
2,2-Dimethyl-3-(2,2-dichlorvinyl)-cyclopropancarbonsäurechlorid,
2,2-Dimethyl-3-(2,2-dibromvinyl)-cyclopropancarbonsäurechlorid,
2,2-Dimethyl-3-(2,2-dimethylvinyl)-cyclopropancarbonsäurechlorid,
α-Isopropyl-phenylessigsäurechlorid,
α-Isopropyl-4-flurophenylessigsäurechlorid,
α-Isopropyl-4-chlorphenylessigsäurechlorid,
α-Isopropyl-4-bromphenylessigsäurechlorid,
α-Isopropyl-4-mehtylphenylessigsäurechlorid,
α-Isopropyl-4-äthylphenylessigsäurechlorid,
α-Isopropyl-4-propylphenylessigsäurechlorid,
α-Isopropyl-4n-iso-propylphenylessigsäurechlorid,
α-Isopropyl-4-flurophenylessigsäurechlorid,
α-Isopropyl-3-bromphenylessigsäurechlorid,
α-Isopropyl-3-chlorphenylessigsäurechlorid,
α-Isopropyl-3-methylphenylessigsäurechlorid,
α-Isopropyl-3-äthylphenylessigsäurechlorid,
α-Isopropyl-3-npropylphenylessigsäurechlorid,
α-Isopropyl-3-iso—propylphenylessigsäurechlorid
und die entsprechenden freien Säuren bzw. deren Natriumsalze.

Die weiterhin als Ausgangsverbindungen zu verwendenden Phenoxybenzylalkohole (III) bzw. Phenoxybenzylhalogenide (V) sind neu, können aber in an sich bekannter Weise erhalten werden, indem man die entsprechenden Phenoxytoluole mit Halogenierungsmitteln, z.B. mit N-Bromsuccinimid in die Phenoxybenzylhalogenide (V) überführt und diese mit Hexamethylentetramin zu den entsprechenden Phenoxybenzaldehyden der Formel (VI)

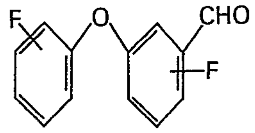
(VI)

unsetzt, und diese

$a^1$) für den Fall, dass $R^1$ für Wasserstoff steht, mit einem komplexen Metallhydrid in einem inerten Lösungsmittel reduziert,

$b^1$) für den Fall, dass $R^1$ für Cyan steht, mit einem Alkalimetallcyanid, z.B. Natrium- oder Kaliumcyanid, in Gegenwart einer Säure gegebenenfalls unter Zusatz eines Lösungsmittels umsetzt oder

$c^1$) für den Fall, dass $R^1$ für Äthinyl steht, mit einer Äthinylverbindung der Formel (VII)

$$HC{\equiv}C{-}MgHal \qquad (VII)$$

in welcher Hal für Halogen steht, in einem geeigneten Lösungsmittel umsetzt.

Verwendet man beispielsweise nach der Verfahrensvariante $a^1$) 5-(3-Fluorphenoxy)-2-fluorbenzaldehyd und Lithiumaluminiumhydrid, nach der Verfahrensvariante $b^1$) 5-(3-Fluorphenoxy)-2-fluor-benzaldehyd und Kaliumcyanid und nach Verfahrensvariante $c^1$) 5-(3-Fluorphenoxy)-2-fluorbenzaldehyd und Äthinylmagnesiumbromid als Ausgangsmaterialien, so kann der Reaktionsverlauf durch die folgenden Formelschemata wiedergegeben werden:

$a^1$) 4×
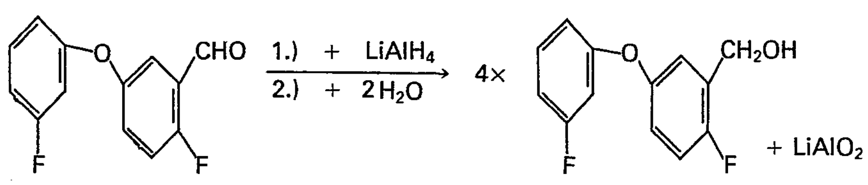


$b^1$)
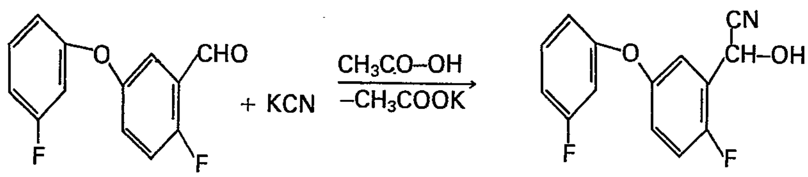


$c^1$)
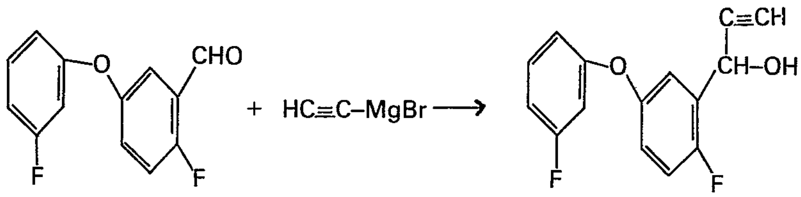


Als Beispiele für die als Ausgangsstoffe zu verwendenden fluorsubstituierten Phenoxybenzylalkohole (III) bzw. –bromide (V) seien im einzelnen genannt:
2-Fluor-3-(2-fluorphenoxy)-benzylalkohol,
4-Fluor-3-(2-fluorphenoxy)-benzylalkohol,
5-Fluor-3-(2-fluorphenoxy)-benzylalkohol,
6-Fluor-3-(2-fluorphenoxy)-benzylalkohol,
2-Fluor-3-(3-fluorphenoxy)-benzylalkohol,
4-Fluor-3-(3-fluorphenoxy)-benzylalkohol,
5-Fluor-3-(3-fluorphenoxy)-benzylalkohol,
6-Fluor-3-(3-fluorphenoxy)-benzylalkohol,

2-Fluor-3-(4-fluorphenoxy)-benzylalkohol,
4-Fluor-3-(4-fluorphenoxy)-benzylalkohol,
5-Fluor-3-(4-fluorphenoxy)-benzylalkohol,
6-Fluor-3-(4-fluorphenoxy)-benzylalkohol,
2-Fluor-3-(2-fluorphenoxy)-α-cyanbenzylalkohol,
4-Fluor-3-(2-fluorphenoxy)-α-cyanbenzylalkohol,
5-Fluor-3-(2-fluorphenoxy)-α-cyanbenzylalkohol,
6-Fluor-3-(2-fluorphenoxy)-α-cyanbenzylalkohol,
2-Fluor-3-(3-fluorphenoxy)-α-cyanbenzylalkohol,
4-Fluor-3-(3-fluorphenoxy)-α-cyanbenzylalkohol,
5-Fluor-3-(3-fluorphenoxy)-α-cyanbenzylalkohol,
6-Fluor-3-(3-fluorphenoxy)-α-cyanbenzylalkohol,
2-Fluor-3-(4-fluorphenoxy)-α-cyanbenzylalkohol,
4-Fluor-3-(4-fluorphenoxy)-α-cyanbenzylalkohol,
5-Fluor-3-(4-fluorphenoxy)-α-cyanbenzylalkohol,
6-Fluor-3-(4-fluorphenoxy)-α-cyanbenzylalkohol,
bzw. die entsprechenden -benzylbromide.

Zur Herstellung der erfindungsgemässen fluorsubstituierten Phenoxybenzyloxycarbonylderivate können als Säureakzeptoren alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines grösseren Bereichs variiert werden. Im allgemeinen arbeitet man zwischen 0 und 150° C, vorzugsweise bei Verfahrensvariante a) zwischen 15 und 40° C und bei Verfahrensvariante b) zwischen 100 und 130° C.

Die Umsetzung lässt man im allgemeinen bei Normaldruck ablaufen.

Die Verfahren zur Herstellung der erfindungsgemässen Verbindungen werden bevorzugt unter Mitverwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol, oder Äther, z.B. Diäthyl- und Dibutyläther, Dioxan, ferner Ketone, beispielsweise Aceton, Methyläthyl-, Methylisopropyl- und Methylisobutylketon, ausserdem Nitrile, wie Aceto- und Propionitril sowie Dimethylformamid.

Zur Durchführung der Verfahrensvariante a) setzt man die Ausgangskomponenten vorzugsweise in äquimolaren Verhältnissen ein. Ein Überschuss der einen oder anderen Komponente bringt keine wesentlichen Vorteile. Die Reaktionskomponenten werden im allgemeinen in einem der angegebenen Lösungsmittel zusammengegeben und meist bei erhöhter Temperatur zur Vervollständigung der Reaktion eine oder mehrere Stunden gerührt. Anschliessend giesst man das Reaktionsgemisch in Wasser, trennt die organische Phase ab und wäscht diese mit Wasser nach. Nach dem Trocknen wird das Lösungsmittel im Vakuum abdestilliert. Bei der Verfahrensvariante b) wird vorzugsweise die Carboxylderivatkomponente in Salzform in einem Lösungsmittel eingesetzt und die Phenoxybenzylbromidkomponente in 10–20%igem Unterschuss zugesetzt. Nach mehrstündigem Erhitzen wird das Lösungsmittel abdestilliert, der Rückstand in einem organischen Lösungsmittel aufgenommen und die organische Phase gewaschen, getrocknet und das Lösungsmittel abdestilliert.

Die neuen Verbindungen fallen in Form von Ölen an, die sich zum Teil nicht unzersetzt destillieren lassen, jedoch durch sogenanntes «Andestillieren», d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

Wie bereits mehrfach erwähnt, zeichnen sich die erfindungsgemässen fluorsubstituierten Phenoxybenzyloxycarbonylderivate durch eine hervorragende insektizide und akarizide Wirksamkeit aus.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpohagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweisshydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige

Polymere verwendet werden, wie Gummiarabicum, Polyvinylalkohol, Polyvinylacetat.

Es können Farbstoffe wie anorganische Pigmente, z.B. Eisenoxyd, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirkstoffe eignen sich auch zur Bekämpfung von Schädlingen auf dem Veterinärsektor.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A

Myzus-Test (Kontakt-Wirkung)

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Kohlpflanzen (Brassica oleracea), welche stark von der Pfirsichblattlaus (Myzus persicae) befallen sind, tropfnass besprüht.

Nach den angegebenen Zeiten wird die Abtötung in % bestimmt. Dabei bedeutet 100%, dass alle Blattläuse abgetötet wurden; 0% bedeutet, dass keine Blattläuse abgetötet wurden.

Bei diesem Test zeigen die Wirkstoffe der Beispiele 1, 3, 4 gute Wirkung.

Beispiel B

Tetranychus-Test (resistent)

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Bohnenpflanzen (Phaseolus vulgaris), die stark von allen Entwicklungsstadien der gemeinen Spinnmilbe oder Bohnenspinnmilbe (Tetranychus urticae) befallen sind, tropfnass besprüht.

Nach den angegebenen Zeiten wird die Abtötung in % bestimmt. Dabei bedeutet 100%, dass alle Spinnmilben abgetötet wurden; 0% bedeutet, dass keine Spinnmilben abgetötet wurden.

Bei diesem Test zeigen die Wirkstoffe der Beispiele 1, 3, 4 gute Wirkung.

Beispiel C

Grenzkonzentrations-Test / Bodeninsekten

Testinsekt: Tenebrio molitor-Larven im Boden

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0 %, wenn noch genau so viele Testinsekten leben wie bei der unbehandelten Kontrolle.

Bei diesem Test zeigten der Wirkstoff des Beispiels 3 eine gute Wirkung.

Beispiel D

Test mit parasitierenden Fliegenlarven (Lucilia cuprina res.)

Emulgator: 80 Gewichtsteile Alkylarylpolyglykoläther

Zur Herstellung einer zweckmässigen Wirkstoffzubereitung vermischt man 20 Gewichtsteile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Grösse beschicktes Teströhrchen gebracht, welches ca. 3 ml einer 20%igen Eigelbpulver-Suspension in Wasser enthält. Auf diese Eigelbpulver-Suspension werden 0,5ml der Wirkstoffzubereitung gebracht. Nach 24 Std. wird der Abtötungsgrad bestimmt.

Bei diesem Test zeigen die Wirkstoffe der Beispiele 3, 4 gute Wirkung.

Beispiel E

Test mit parasitierenden adulten Rinderzecken (Boophilus microplus res.)
Lösungsmittel: Alkylarylpolyglykoläther

Zur Herstellung einer zweckmässigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (B. microplus res.) werden in der zu testenden Wirkstoffzubereitung 1 Min. getaucht. Nach Überführung in Plastikbecher und Aufbewahrung in einem klimatisierten Raum wird der Abtötungsgrad bestimmt.

Bei diesem Test zeigen die Wirkstoffe der Beispiele 3, 4 gute Wirkung.

Beispiel F

$LD_{100}$-Test
Testtiere: Sitophilus granarius
Lösungsmittel: Aceton

2 Gewichtsteile Wirkstoff werden in 1000 Volumenteilen Lösungsmittel aufgenommen. Die so erhaltene Lösung wird mit weiteren Lösungsmitteln auf die gewünschten Konzentrationen verdünnt.

2,5ml Wirkstofflösung werden in eine Petrischale pipettiert. Auf dem Boden der Petrischale befindet sich ein Filterpapier mit einem Durchmesser von etwa 9,5cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man etwa 25 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Bei diesem Test zeigen die Wirkstoffe der Beispiele 1, 3 und 4 gute Wirkung.

Herstellungsbeispiele

Beispiel 1:

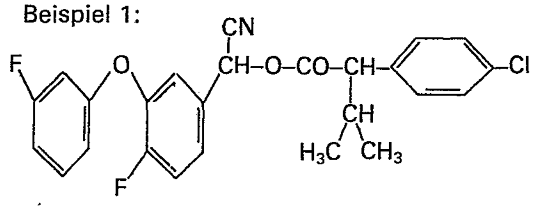

6,5g (0,025 Mol) 3-(3-Fluorphenoxy)-4-fluor-α-cyanbenzylalkohol und 5,8g (0,025 Mol) α-Isopropyl-4-chlorphenylessigsäurechlorid werden in 150ml wasserfreiem Toluol gelöst und bei 25–30° C 2,4 g (0,03 Mol) Pyridin, gelöst in 50ml Toluol, unter Rühren zugetropft. Anschliessend wird weitere 3 Stunden bei 25° C gerührt. Das Reaktionsgemisch wird in 150ml Wasser gegossen, die organische Phase abgetrennt und mit 100ml Wasser gewaschen. Anschliessend wird die Toluolphase über Natriumsulfat getrocknet und das Lösungsmittel im Wasserstrahlvakuum abdestilliert. Letzte Lösungsmittelreste werden durch kurzes Andestillieren bei 70° C/1,33 · $10^{-3}$ bar Badtemperatur entfernt. Man erhält 7,5g (65,9% der Theorie) 3-(3-Fluorphenoxy)-4-fluor-α-cyanbenzyl-α'-isopropyl-4'-chlorphenylacetat als gelbes Öl mit dem Berechnungsindex $n_D^{25}$: 1,5418.

Beispiel 2:

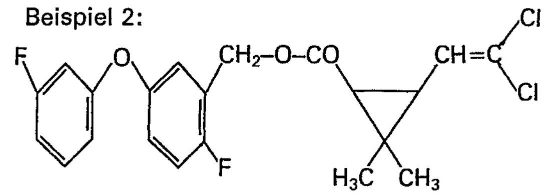

6,9 g (0,03 Mol) 2,2-Dimethyl-3-(2,2-dichlorvinyl)-cyclopropancarbonsäure-natriumsalz werden in 150ml Dimethylformamid gelöst und zusammen mit 8,0g (0,027 Mol) 3-(3-Fluorphenoxy)-6-fluor-benzylbromid 4 Stunden auf 120° C erhitzt. Nach beendeter Reaktion wird das Dimethylformamid im Vakuum abdestilliert und der verbleibende Rückstand in 200ml Methylenchlorid aufgenommen. Anschliessend wird zweimal mit je 150ml Wasser ausgeschüttelt, die organische Phase über Natriumsulfat getrocknet und das Lösungsmittel im Vakuum abgezogen. Letzte Lösungsmittelreste werden durch kurzes Andestillieren bei 70° C/1,333 · $10^{-3}$ bar Badtemperatur entfernt. Man erhält 8,0g (71% der Theorie) 3-(3-Fluorphenoxy)-6-fluor-benzyl-2,2-dimethyl-3(2,2-dichlorvinyl)-cyclopropancarboxylat als gelbes Öl mit dem Berechnungsindex $n_D^{24}$: 1,5395.

In analoger Weise können die folgenden Verbindungen der folgenden Beispiele dargestellt werden:

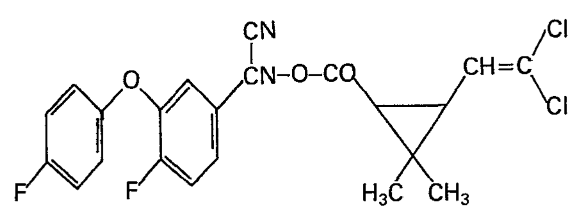

Brechungsindex
$n_D^{26}$:1,5326

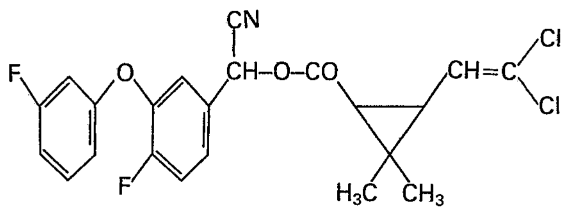

Brechungsindex
$n_D^{25}$: 1,5375

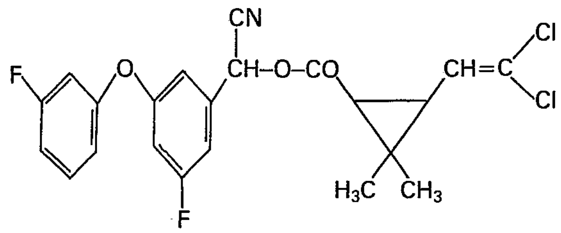

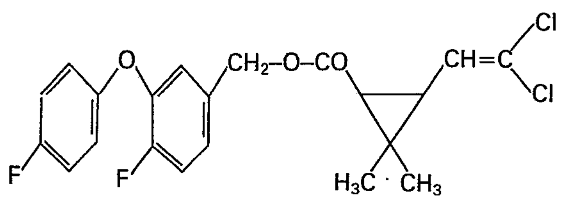

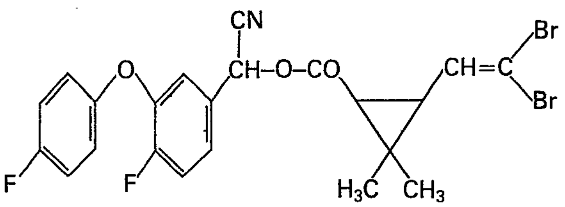

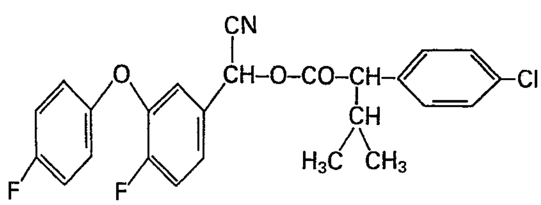

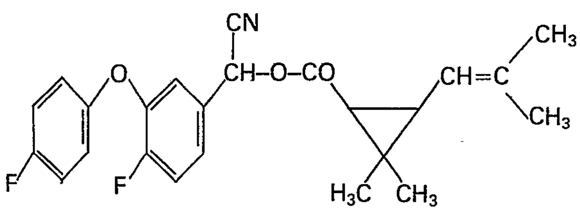

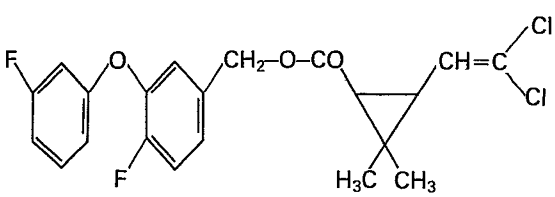

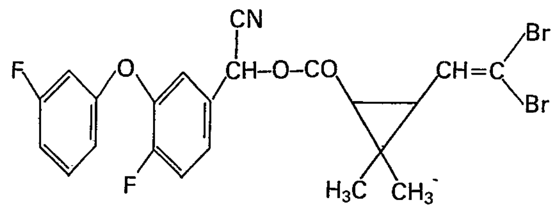

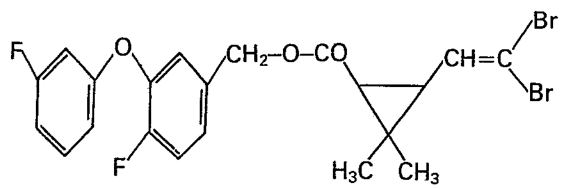

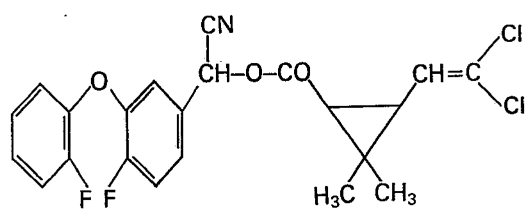

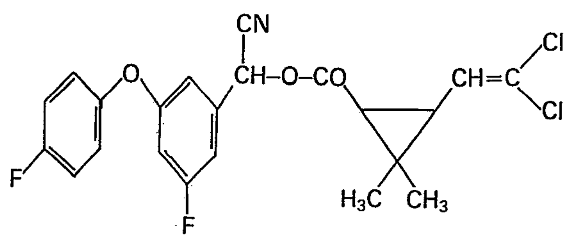

Die als Ausgangsverbindungen benötigten Pheonoxybenzylbromide bzw. Phenoxybenzylalkohole können wie im folgenden beschrieben hergestellt werden:

a) 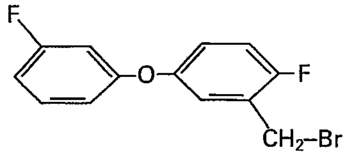

48,4 g (0,22 Mol) 3-(3-Fluorphenoxy)-6-fluor-toluol werden in 300 ml wasserfreiem Tetrachlor-

kohlenstoff gelöst und zusammen mit 41g N–Bromsuccinimid am Rückfluss erhitzt. Nach Erreichen von 70° C werden 3g Azodiisobuttersäurenitril zugesetzt, nach ca. 10–20 Minuten setzt die Reaktion unter Wärmeentwicklung ein und nach Abklingen der exothermen Reaktion wird noch 4 Stunden am Rückfluss erhitzt. Der Reaktionsansatz wird dann auf 10° C abgekühlt, das Succinimid abgesaugt und der Tetrachlorkohlenstoff im Vakuum abdestilliert. Das verbleibende Öl wird bei 142–150° C/2,666 · $10^{-3}$ bar destilliert. Man erhält in 51%iger Ausbeute 3-(3-Fluorphenoxy)-6-fluor-benzylbromid.

Analog können dargestellt werden:

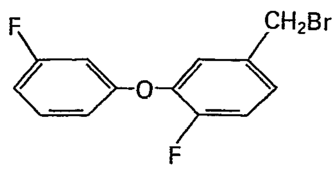

Siedepunkt 140–150° C/1,333 · $10^{-3}$bar
Ausbeute: 45% der Theorie

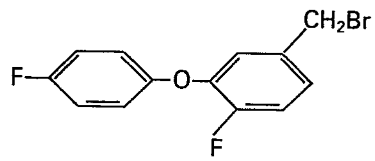

Siedepunkt 142–151° C/2,666 · $10^{-3}$bar
Ausbeute: 47% der Theorie

b) 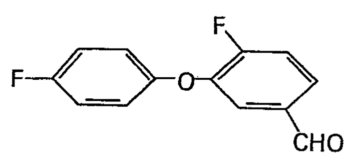

56g (0,187 Mol) 3-(4-Fluorphenoxy)-4-fluorbenzylbromid und 53g Hexamethylentetramin in 500ml Petroläther werden 3 Stunden am Rückfluss erhitzt, anschliessend wird auf 5–10° C abgekühlt und der entstandene Niederschlag abgesaugt. Dieser wird mit 100ml Petroläther gewaschen, trockengesaugt und dann in 100ml 50%iger wässriger Essigsäure 5 Stunden am Rückfluss erhitzt. Danach fügt man 25ml konzentrierte Salzsäure hinzu, erhitzt nochmals 30 Minuten am Rückfluss und kühlt anschliessend auf 10–20° C ab. Das Reaktionsgemisch wird mit 200ml Wasser versetzt und zweimal mit je 150ml Äther extrahiert und die vereinigten Ätherphasen anschliessend mit Natriumhydrogencarbonatlösung gewaschen und über Natriumsulfat getrocknet. Der Äther wird im Vakuum abdestilliert. Man erhält in 40%iger Ausbeute 3-(4-Fluorphenoxy)-4-fluor-benzaldehyd mit dem Siedepunkt von 133–135° C/1,333 · $10^{-3}$bar.

Analog lässt sich darstellen:

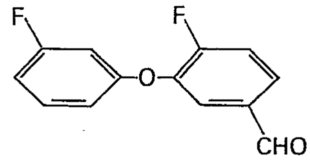

Kochpunkt 138–139° C/2,666 · $10^{-3}$bar
Ausbeute: 46% der Theorie

c₁) 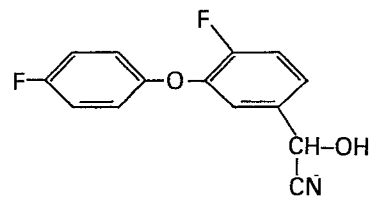

9,6g (0,041 Mol) 3-(4-Fluorphenoxy)-4-fluorbenzaldehyd werden in 50ml Eisessig gelöst und bei 15° C unter Rühren 9g Natriumcyanid, gelöst in 50ml Wasser, zugetropft. Anschliessend wird 8 Stunden bei 20° C gerührt, das Reaktionsgemisch in 100ml Wasser gegossen, mit 200ml Äther extrahiert und die Ätherphase mit verdünnter Natriumbicarbonatlösung gewaschen und dann über Natriumsulfat getrocknet. Nach dem Abdestillieren des Äthers im Vakuum erhält man in 90%iger Ausbeute 3-(4-Fluorphenoxy)-4-fluor-α-cyanbenzylalkohol mit dem Brechungsindex $n_D^{24}$: 1,5643.

Analog lässt sich darstellen:

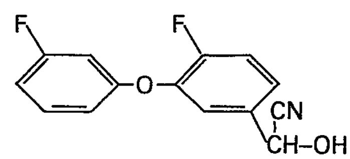

Brechungsindex: $n_D^{24}$: 1,5591
Ausbeute: 91% der Theorie

c₂) 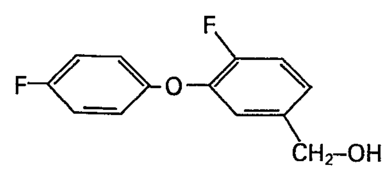

Zu 3,8g Lithiumaluminiumhydrid in 100ml wasserfreiem Äther werden in der Siedehitze 58,5g (0,25 Mol) 3-(4-Fluorphenoxy)-4-fluor-benzaldehyd, gelöst in 50ml trockenem Äther, unter gutem Rühren zugetropft. Anschliessend wird 10 Stunden bei 22° C nachgerührt, der Reaktionsansatz dann auf 0° C abgekühlt und unter Rühren solange Eiswasser zugetropft, bis keine Wasserstoffentwicklung mehr zu beobachten ist. Der entstandene Niederschlag wird durch Zusatz von 10%iger Schwefelsäure gelöst und anschliessend das Reaktionsgemisch 2 mal mit je 100ml Äther extrahiert. Die Ätherphasen werden abgetrennt, mit gesättigter Kochsalzlösung gewaschen und über Natriumsulfat getrocknet. Nach dem Abdestillieren des Äthers im Vakuum erhält man in 80%iger Ausbeute 3-(4-Fluorphenoxy)-4-fluorbenzylalkohol mit dem Siedepunkt 155–160° C/2,666 · $10^{-3}$bar.

**Patentansprüche**

1) Fluorsubstituierte Phenoxybenzyl-oxycarbonylderivate, -alkohole und -halogenide der Formel (I)

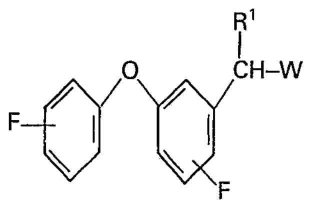

(I)

in welcher

W für OH, Halogen oder den Rest $-OCO-R^2$ steht und $R^1$ für Wasserstoff, Cyan oder Äthinyl steht und

$R^2$ für den Rest

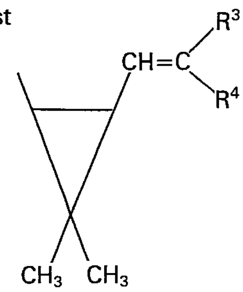

steht, wobei

$R^3$ und $R^4$ gleich sind und für Chlor, Brom oder Methyl stehen und ferner

$R^2$ für den Rest

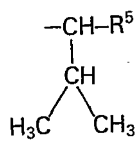

steht, wobei

$R^5$ für einen gegebenenfalls durch Halogen, Alkyl, Alkylthio bzw. Alkoxy mit jeweils 1–4 Kohlenstoffatomen, Nitro oder Methylendioxy substituierten Phenylring steht.

2) Verfahren zur Herstellung der fluorsubstituierten Phenoxybenzyl-oxycarbonylderivate, -alkohole und -halogenide der Formel (I)

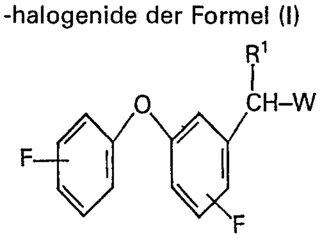

(I)

in welcher

W für OH, Halogen oder den Rest $-OCO-R^2$ steht und

$R^1$ für Wasserstoff, Cyan oder Äthinyl steht und

$R^2$ für den Rest

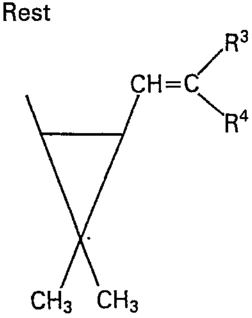

steht, wobei

$R^3$ und $R^4$ gleich sind und für Chlor, Brom oder Methyl stehen und ferner

$R^2$ für den Rest

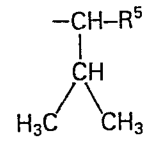

steht, wobei

$R^5$ für einen gegebenenfalls durch Halogen, Alkyl, Alkylthio bzw. Alkoxy mit jeweils 1–4 Kohlenstoffatomen, Nitro oder Methylendioxy substituierten Phenylring steht, gemäss Anspruch 1, das dadurch gekennzeichnet ist, dass man für den Fall, dass W für den Rest $-O-CO-R^5$ steht,

a) Carbonylhalogenide der Formel (II)

$$Hal-CO-R^2 \qquad (II)$$

in welcher

$R^2$ die oben angegebene Bedeutung hat, und Hal für Halogen, vorzugsweise Chlor, steht, mit fluorsubstituierten Phenoxybenzylalkoholen der Formel (III)

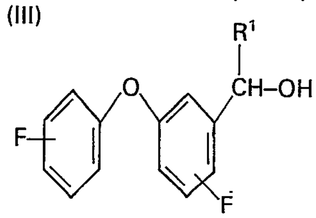

(III)

in welcher

$R^1$ die oben angegebene Bedeutung hat, gegebenenfalls in Gegenwart eines Säureakzeptors und gegebenenfalls in Gegenwart eines Lösungsmittels umsetzt, oder

b) Carboxylderivate der Formel (IV)

$$HO-CO-R^2 \qquad (IV)$$

in welcher

$R^2$ die angegebene Bedeutung hat, gegebenenfalls in Form der Alkali-, Erdalkali- oder Ammoniumsalze oder gegebenenfalls in Gegenwart eines Säureakzeptors mit fluorsubstituierten Phenoxybenzylhalogeniden der Formel (V)

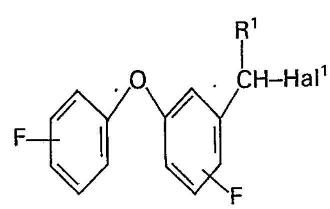

(V)

in welcher

$R^1$ die angegebene Bedeutung hat, und $Hal^1$ für Halogen, vorzugsweise Brom, steht, gegebenenfalls in Gegenwart eines Lösungsmittels umsetzt, und ferner für den Fall, dass W für OH oder Halogen steht, in an sich bekannter Weise die entsprechenden Phenoxytoluole mit Halogenierungsmitteln in die entsprechenden Phenoxybenzylhalogenide überführt, diese mit Hexamethylentetramin zu den entsprechenden Phenoxybenzaldehyden umsetzt und diese

a') für den Fall, dass R' für Wasserstoff steht, mit einem komplexen Metallhydrid in einem inerten Lösungsmittel reduziert,

b') für den Fall, dass R' für Cyan steht, mit einem Alkalimetallcyanid in Gegenwart einer Säure gegebenenfalls unter Zusatz eines Lösungsmittels umsetzt, oder

c') für den Fall, dass R' für Äthinyl steht, mit einer Äthinylverbindung der Formel

$$H–C{\equiv}Mg–Hal$$

in welcher Hal für Halogen steht in einem geeigneten Lösungsmittel umsetzt.

3) Insektizide und akarizide Mittel gekennzeichnet durch einen Gehalt an mindestens einem fluorsubstituierten Phenoxybenzyloxycarbonylderivat der Formel (I), in welcher W für den Rest $-OCO-R^2$ steht, gemäss Anspruch 1.

4) Verwendung von fluorsubstituierten Phenoxybenzyloxycarbonylderivaten der Formel I, in welcher W für den Rest $-OCOR^2$ steht, gemäss Anspruch 1, zur Bekämpfung von Insekten oder Acarinae.

5) Verfahren zur Herstellung insektizider oder akarizider Mittel, dadurch gekennzeichnet, dass man fluorsubstituierte Phenoxybenzyloxycarbonylderivate der Formel I, in welcher W für $-OCOR^2$ steht, gemäss Anspruch 1, mit Streckmittel und/oder oberflächenaktiven Stoffen vermischt.

**Revendications**

1. Dérivés phénoxybenzyloxycarbonylés, alcools et halogénures fluorosubstitués de formule

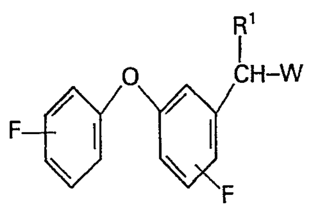

(I)

dans laquelle W représente OH, un halogène ou le reste $O-CO-R^2$ et $R^1$ représente l'hydrogène, un groupe cyan ou éthynyle et

$R^2$ représente le reste

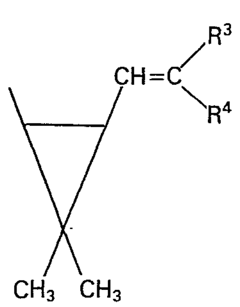

où $R^3$ et $R^4$ sont identiques et représentent le chlore, le brome ou un méthyle, et en outre

$R^2$ représente le reste

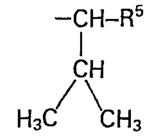

où $R^5$ représente un noyau phényle éventuellement substitué par un halogène, un alkyle, un alkylthio ou alcoxy en $C_1-C_4$, un nitro ou en méthylènedioxy.

2. Procédé pour la préparation de dérivés phénoxybenzyloxycarbonylés, alcools et halogénures fluorosubstitués de formule

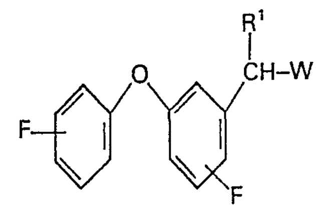

(I)

dans laquelle W représente OH, un halogène ou le reste $O-CO-R^2$ et $R^1$ représente l'hydrogène, un groupe cyan ou éthynyle et

$R^2$ représente le reste

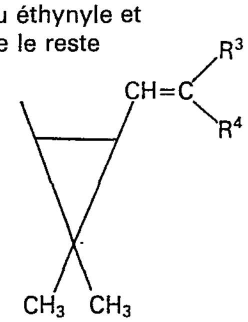

où $R^3$ et $R^4$ sont identiques et représentent le chlore, le brome ou en méthyle et, en outre,

$R^2$ représente le reste

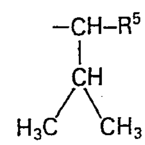

où $R^5$ représente un noyau phényle éventuellement substitué par un halogène, un alkyle, alkylthio ou alcoxy en $C_1-C_4$ ou un méthylènedioxy, caractérisé en ce que, dans le cas où W représente le reste $-O-CO-R^2$,

a) on fait réagir des halogénures de carbonyle de formule (II)

$$Hal-CO-R^2 \qquad (II)$$

dans laquelle $R^2$ a la signification indiquée et Hal représente un halogène, avec des alcools phénoxybenzyliques fluorsubstitués de formule

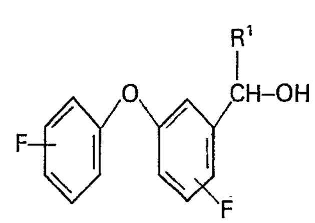

(III)

dans laquelle $R^1$ a la signification indiquée, éventuellement en présence d'un accepteur d'acide et éventuellement en présence d'un solvant, ou bien

b) on fait réagir des dérivés carboxylés de formule

$$HO\text{–}CO\text{–}R^2 \qquad (IV)$$

dans laquelle $R^2$ a la signification indiquée, éventuellement sous forme des sels alcalins, alcalino-terreux ou d'ammonium ou éventuellement en présence d'un accepteur d'acide, avec des halogénures de phénoxybenzyle fluorsubstitués de formule

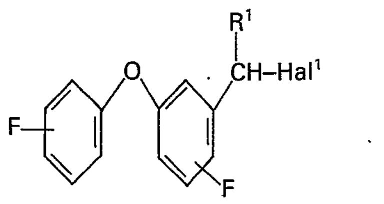

(V)

dans laquelle $R^1$ a la signification indiquée, et $Hal^1$ représente un halogène, éventuellement en présence d'un solvant, et, en outre, dans le cas où W représente OH ou un halogène, ou transforme de manière connue les phénoxytoluènes correspondants avec des agents halogénants en les halogénures de phénoxybenzyle correspondants, on fait réagir ceux-ci avec l'hexaméthylènetétramine en les phénoxybenzaldéhydes correspondants et

a') dans le cas où $R^1$ représente l'hydrogène, on réduit ces derniers par un hydrure métallique complexe dans un solvant inerte,

b') dans le cas où $R^1$ représente un groupe cyano, on fait réagir ces derniers avec un cyanure de métal alcalin en présence d'un acide, éventuellement avec addition d'un solvant, ou bien

c') dans le cas où $R^1$ représente le groupe éthynyle, on fait réagir ces derniers avec un composé éthynylé de formule

$$H\text{–}C{\equiv}Mg\text{–}Hal$$

dans laquelle Hal représente un halogène, dans un solvant approprié.

3. Agents insecticides et acaricides, caractérisés en ce qu'ils contiennent au moins un dérivé phénoxybenzyloxycarbonylé fluorosubstitué de formule I, dans laquelle W représente le reste $-OCO-R^2$, selon la revendication 1.

4. Utilisation des dérivés phénoxybenzyloxycarbonylés fluorosubstitués de formule I, dans laquelle W représente le reste $-OCOR^2$, selon la revendication 1, pour la lutte contre les insectes ou les araignées.

5. Procédé pour la préparation d'agents insecticides ou acaricides, caractérisé en ce que l'on mélange des dérivés phénoxybenzoyloxycarbonylés fluorsubstitués de formule I dans laquelle W représente $-OCOR^2$, selon la revendication 1, avec des agents diluants et/ou des substances.

**Claims**

1) Fluorine-substituted phenoxybenzyl-oxycarbonyl derivates, alcohols and halides of the formula (I)

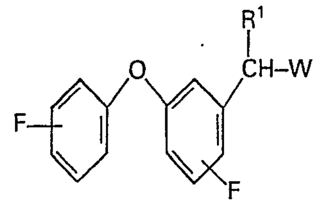

(I)

in which

W represents OH, halogen or the radical $-OCO-R^2$ and

$R^1$ represents hydrogen, cyano or ethinyl and

$R^2$ represents the radical

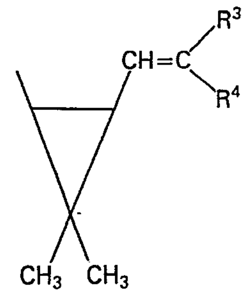

wherein

$R^3$ and $R^4$ are identical and represent chlorine, bromine or methyl, and furthermore $R^2$ represents the radical

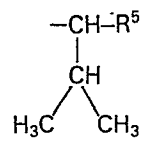

wherein

$R^5$ represents a phenyl ring which is optionally substituted by halogen, alkyl, alkylthio or alkoxy with in each case 1–4 carbon atoms, nitro or methylenedioxy.

2) Process for the preparation of the fluorinesubstituted phenoxybenzyl-oxycarbonyl derivatives, alcohols and halides of the formula (I)

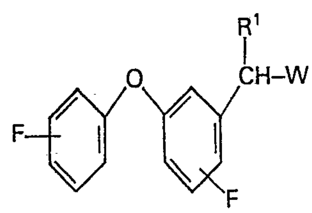

(I)

in which

W represents OH, halogen or the radical $-OCO-R^2$ and

$R^1$ represents hydrogen, cyano or ethinyl and $R^2$ represents the radical

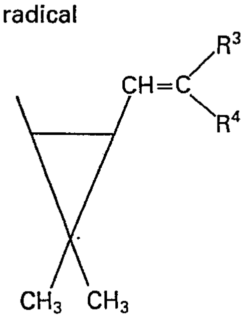

wherein

$R^3$ and $R^4$ are identical and represent chlorine, bromine or methyl, and furthermore $R^2$ represents the radical

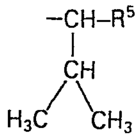

wherein

$R^5$ represents a phenyl ring which is optionally substituted by halogen, alkyl, alkylthio or alkoxy with in each case 1–4 carbon atoms, nitro or methylenedioxy, which is characterised in that in the case where W represents the radical $-O-CO-R^2$

a) carbonyl halides of the formula (II)

$$Hal-CO-R^2 \quad (II)$$

in which

$R^2$ has the meaning given and Hal represents halogen, preferably chlorine, are reacted with fluorine-substituted phenoxybenzyl alcohols of the formula (III)

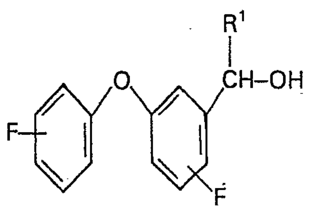

(III)

in which

$R^1$ has the meaning given, if appropriate in the presence of an acid acceptor and if appropriate in the presence of a solvent, or

b) carboxyl derivatives of the formula (IV)

$$HO-CO-R^2 \quad (IV)$$

in which

$R^2$ has the meaning given, if appropriate in the form of the alkali metal salts, alkaline earth metal salts of ammonium salts or, if appropriate, in the presence of an acid acceptor, are reacted with fluorine-substituted phenoxybenzyl halides of the formula (V)

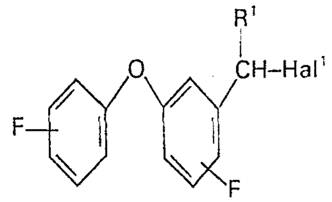

(V)

in which

$R^1$ has the meaning given and

$Hal^1$ represents halogen, preferably bromine, if appropriate in the presence of a solvent, and furthermore, in the case where W represents OH or halogen, the corresponding phenoxytoluenes are converted into the corresponding phenoxybenzyl halides with halogenating agents in a manner which is in itself known, these halides are reacted with hexamethylenetetramine to give the corresponding phenoxybenzaldehydes and these phenoxybenzaldehydes

$a^1$) in the case where $R^1$ represents hydrogen, are reduced with a complex metal hydride in an inert solvent, or

$b^1$) in the case where $R^1$ represents cyano, are reacted with an alkali metal cyanide in the presence of an acid, if appropriate with the addition of a solvent, or

$c^1$) in the case where $R^1$ represents ethinyl, are reacted with an ethinyl compound of the formula

$$H-C \equiv C-Mg-Hal$$

in which

Hal represents halogen, in a suitable solvent.

3) Insecticidal and acaricidal agents, characterised in that they contain at least one fluorine-substituted phenoxybenzyl-oxycarbonyl derivative of the formula (I), in which W represents the radical $-OCO-R^2$, according to Claim 1.

4) Use of fluorine-substituted phenoxybenzyl-oxycarbonyl derivatives of the formula I, in which W represents the radical $-OCOR^2$, according to Claim 1, for combating insects or Acarinae.

5) Process for the preparation of insecticidal or acaricidal agents, characterised in that fluorinesubstituted phenoxybenzyl-oxycarbonyl derivatives of the formula I, in which W represents $-OCOR^2$, according to Claim 1, are mixed with extenders and/or surface-active substances.